(19)

Europäisches
Patentamt
European
Patent Office
Office européen
des brevets

(11) **EP 2 589 429 A1**

(12) **EUROPEAN PATENT APPLICATION**

(43) Date of publication:
**08.05.2013 Bulletin 2013/19**

(51) Int Cl.:
**B01J 19/00** *(2006.01)*

(21) Application number: **12188375.5**

(22) Date of filing: **12.10.2012**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br><br>(30) Priority: **14.10.2011 SE 1150954**<br><br>(71) Applicant: **Tekniska Verken I Linköping AB**<br>**581 15 Linköping (SE)** | (72) Inventors:<br>• **Nordell, Erik**<br>**582 34 Linköping (SE)**<br>• **Moestedt, Jan**<br>**602 24 Norrköping (SE)**<br>• **Karlsson, Martin**<br>**585 99 Linköping (SE)**<br><br>(74) Representative: **Simonsson, Erik**<br>**Awapatent AB**<br>**P.O. Box 99**<br>**351 04 Växjö (SE)** |

(54) **Biogas producing laboratory reactor and method**

(57)     A biogas producing laboratory reactor (1) comprises a cavity (8) for containing organic material during the digestion thereof, and a level setting device (28). The level setting device (28) comprises a drain opening (60) which is fluidly connected to a drain pipe (48) and through which material may be drained from the cavity (8) and into the drain pipe (48). The level setting device (28) further comprises a lower flow restriction (64) which sets a vertical level of material inside of the cavity (8) and over which the organic material must flow when passing from the drain opening (60) and into the drain pipe (48). The lower flow restriction (64) is located on a higher vertical level than the drain opening (60). A method of performing a laboratory experiment of digesting organic material in said biogas producing laboratory reactor (1), the method comprising: feeding organic material to a cavity (8) of the laboratory reactor (1), digesting the organic material inside the cavity (8) under anaerobic conditions with production of biogas, and adjusting the amount of material in the cavity (8) by a level setting device (28).

Fig. 1

EP 2 589 429 A1

**Description**

<u>Field of the Invention</u>

The present invention relates to a biogas producing laboratory reactor comprising a cavity for containing organic material during the digestion thereof, and a level setting device comprising a drain pipe via which organic material contained inside of the cavity can be drained out of the cavity.

The present invention further relates to a method of performing a laboratory experiment of digesting organic material in a biogas producing laboratory reactor.

<u>Background of the Invention</u>

Bio-reactors are frequently utilized for digesting organic material, such as agricultural products, food waste, sewage sludge, etc. for the purpose of reducing the volume of the organic material, and producing biogas. A full-scale bio-reactor may typically contain a volume of 1000 - 10 000 m$^3$ of organic material undergoing digestion. With such large volumes of organic material, experiments made to optimize and develop processes become expensive and risky. Hence, there is a need for a device making it possible to perform small scale experiments at relevant operating conditions, prior to implementing new operating principles, new organic materials, new additives, etc. in a full-scale reactor.

DE 40 06 382 A1 discloses a laboratory reactor which may be utilized for making small scale experiments. However, experiments performed in the laboratory reactor of DE 40 06 382 A1 do not give sufficiently accurate experimental results for being safely applied to operation in a full-scale bio-reactor.

<u>Summary of the Invention</u>

An object of the present invention is to provide a laboratory reactor in which reliable small scale experiments with digestion of organic material can be performed.

This object is achieved by a biogas producing laboratory reactor comprising a cavity for containing organic material during the digestion thereof, and a level setting device comprising a drain pipe via which organic material contained inside of the cavity can be drained out of the cavity. The level setting device further comprises a drain opening which is fluidly connected to the drain pipe and through which material may be drained from the cavity and into the drain pipe, and a lower flow restriction which sets a vertical level of material inside of the cavity and over which the organic material must flow when passing from the drain opening and into the drain pipe, wherein the lower flow restriction is located on a higher vertical level than the drain opening.

An advantage of this laboratory reactor is that organic material can be drained from the cavity of the reactor with high precision both as regards the level of material remaining in the cavity, and as regards the particle size distribution of the material being drained from the cavity in relation to the particle size distribution of the bulk of material in the cavity. Thereby accurate laboratory experiments for evaluation of biogas production can be conducted, which improves the possibility to apply, in a reliable and predictable manner, the results of such laboratory experiments to biogas production of a full-scale plant.

According to one embodiment the lower flow restriction is located at a vertical distance of 5-300 mm above the position of the drain opening. An advantage of this embodiment is that a material which is representative of the bulk of material of the cavity can be drained, without generating unduly long paths of transport of the material to be drained.

According to one embodiment the lower flow restriction is located at an intended liquid height above an inner bottom of the cavity, wherein a vertical distance between the position of the drain opening and the lower flow restriction amounts to 1-40 % of the intended liquid height. An advantage of this embodiment is that the drain opening is always located below the liquid surface of the material, but still not too close to the bottom, where a collection of non-representative material could occur.

According to one embodiment the level setting device further comprises a ventilation opening for fluidly connecting the interior of the level setting device to a gas space for preventing a siphoning effect in the level setting device. An advantage of this embodiment is that problems related to unwanted suction of material from the cavity can be avoided or at least reduced.

According to one embodiment the drain opening faces downwards. An advantage of this embodiment is that collecting a representative material from the bulk of material of the cavity is made easier and more accurate.

According to one embodiment the drain opening and the drain pipe are fluidly connected to each other by means of a tube bend. An advantage of this embodiment is that an efficient, yet simple, level setting device is obtained, which has a low risk of material being clogged inside of it.

According to one embodiment the drain opening and the drain pipe are fluidly connected to each other by means of a branch tube. An advantage of this embodiment is that an efficient design is obtained, which can also be adapted to situations where the level of material inside of the cavity varies within a wide range.

According to one embodiment the laboratory reactor comprises a gas buffering system having an expandable gas bag and a gas pipe connecting the gas bag to a biogas reactor headspace of the cavity, the gas buffering system being adapted to transport biogas contained in the expandable gas bag to the biogas reactor headspace of the cavity when adjusting the volume of organic material contained in the cavity by means of the level setting device. An advantage of this embodiment is that the level of material can be adjusted without causing a change in the pressure inside of the cavity, and without causing air to be drawn into the cavity.

According to one embodiment, the laboratory reactor further comprises an analyzing instrument for measuring the amount of biogas produced. According to a further embodiment, an analyzing instrument measures the composition of the produced biogas.

According to one embodiment the total volume of the cavity is 1-100 litres. A volume of less than 1 litre has been found to make it difficult to obtain accurate experimental results in the laboratory reactor. A volume of more than 100 litres makes the laboratory experiments more costly and manpower demanding, without further increasing the accuracy of the experimental test results.

According to one embodiment the drain pipe extends downwards from the lower flow restriction. An advantage of this embodiment is that material may flow over the lower flow restriction, into the drain pipe and out of the cavity by its own weight, i.e., by the force of gravity. Hence, a pump for pumping material out of the cavity may be dispensed of.

According to one embodiment the laboratory reactor further comprises a feeding device comprising a feeding tube adapted to extend below a liquid surface of the organic material contained in the cavity. An advantage of this embodiment is that newly fed material may be quickly immersed into and mixed with the bulk of material in the cavity, and can be accessible for digestion almost immediately after being fed to the cavity.

According to one embodiment the feeding device comprises a plunger for forcing organic material fed to the feeding tube into the bulk of organic material. An advantage of this embodiment is that material may be more efficiently forced into the bulk of material in the cavity, to obtain quick mixing.

According to one embodiment, the plunger of the feeding device may be provided with a notch extending along the length of the plunger, to enable air of the feeding tube to escape when the plunger is utilized for supplying material to the bulk of organic material in the cavity. An advantage of this embodiment is that it reduces the risk that air is inadvertently injected into the cavity while feeding material to be digested.

According to one embodiment a lower end of the plunger extends below a lower end of the feeding tube. Thereby, biological material to be fed to the cavity is efficiently mixed with the bulk of biological material present inside the cavity.

According to one embodiment, the cavity comprises a single biogas reactor headspace. An advantage of this embodiment is that most or even all of the gas that is produced by the material is collected in one place.

A further object of the present invention is to provide an efficient method of performing a laboratory experiment of digesting organic material.

This object is achieved by means of a method of performing a laboratory experiment of digesting organic material in a biogas producing laboratory reactor, the method comprising:

feeding organic material to a cavity of the laboratory reactor,
digesting the organic material inside the cavity under anaerobic conditions with production of biogas, and
adjusting the amount of material in the cavity by a level setting device comprising a drain pipe via which material contained inside of the cavity can be drained out of the cavity, such material being drained out of the cavity via a drain opening which is fluidly connected to the drain pipe until a vertical level of material inside of the cavity reaches a lower flow restriction which is located on a higher vertical level than the drain opening and over which the organic material must flow when passing from the drain opening and into the drain pipe.

An advantage of this method is that laboratory experiments can be carried out with high precision, such that the experimental results can be reliably applied to operation of full scale bio-reactors.

According to one embodiment the method further comprises transporting biogas contained in an expandable gas bag to a biogas reactor headspace of the cavity when adjusting the amount of organic material contained in the cavity by means of the level setting device. An advantage of this embodiment is that the pressure level inside of the cavity can be kept almost constant, and that air can be prevented from leaking into the cavity.

According to one embodiment, the method comprises measuring the amount of biogas produced. An advantage of this embodiment is that accurate, and optionally on-line, evaluation of the biogas production in the laboratory experiment can be obtained. According to a further embodiment the composition of the produced biogas is measured. Thereby, the quality of the produced biogas can be evaluated.

According to one embodiment the method further comprises feeding organic material to be digested to the cavity via a feeding tube extending below a liquid surface of the organic material contained in the cavity. An advantage of this embodiment is that material to be digested can be fed to the cavity without causing air to leak into a biogas reactor headspace of the cavity.

According to one embodiment the method further comprises utilizing a plunger for forcing organic material fed to the feeding tube into the bulk of organic material. An advantage of this embodiment is that newly fed material is quickly immersed into and mixed with the bulk of material in the cavity, and can be accessible for digestion almost immediately after being fed to the cavity.

According to one embodiment the method comprises transporting biogas contained in a biogas reactor headspace of the cavity to an expandable gas bag when feeding organic material to the cavity. An advantage of this embodiment is that the pressure inside the cavity remains constant also after supplying material to be digested to the cavity. Furthermore, the biogas can be stored in the expandable gas bag and then transported back to the biogas reactor headspace of the cavity when needed due to a reduction in the volume of organic material present in the cavity caused by digestion of the material or caused by draining material from the cavity.

According to one embodiment the method further comprises ventilating the interior of the level setting device to a gas space for preventing a siphoning effect in the level setting device. An advantage of this embodiment is that the draining of material from the cavity to yield a certain desired level of the liquid surface in the cavity becomes even more accurate.

According to one embodiment the method comprises performing the laboratory experiment at a dry solids content of the biological material in the cavity of 1-20 % by weight DS, preferably 3-12 % by weight DS. These dry solids contents in the cavity yields efficient digestion of biological material.

According to one embodiment material is drained out of the cavity under the force of gravity. An advantage of this embodiment is that a pump for pumping material out of the cavity may be dispensed of, since the material flows, by its own weight, out of the cavity via the level setting device.

Further objects and features of the present invention will be apparent from the following detailed description and claims.

Brief description of the Drawings

The invention is described in more detail below with reference to the appended drawings in which:

Fig. 1 is a schematic side view of a biogas producing laboratory reactor in accordance with a first embodiment.
Fig. 2 is a schematic perspective view of a level setting device according to prior art.
Fig. 3 is a schematic side view of a level setting device according to a first embodiment.
Fig. 4a is a schematic side view of a level setting device according to a second embodiment.
Fig. 4b is a schematic side view of a level setting device according to a third embodiment.
Fig. 5a is a schematic side view of a gas buffering system in a first mode of operation.
Fig. 5b is a schematic side view of a gas buffering system in a second mode of operation.
Fig. 6 is a schematic side view of a feeding device of the biogas producing laboratory reactor.
Fig. 7a is a schematic side view of a plunger of the feeding device of Fig. 6.
Fig. 7b is a schematic side view of the plunger of Fig. 7a when being tightened to a feeding tube.
Fig. 7c is a schematic side view of the plunger of Fig. 7a in a sealing position.

Description of Preferred Embodiments

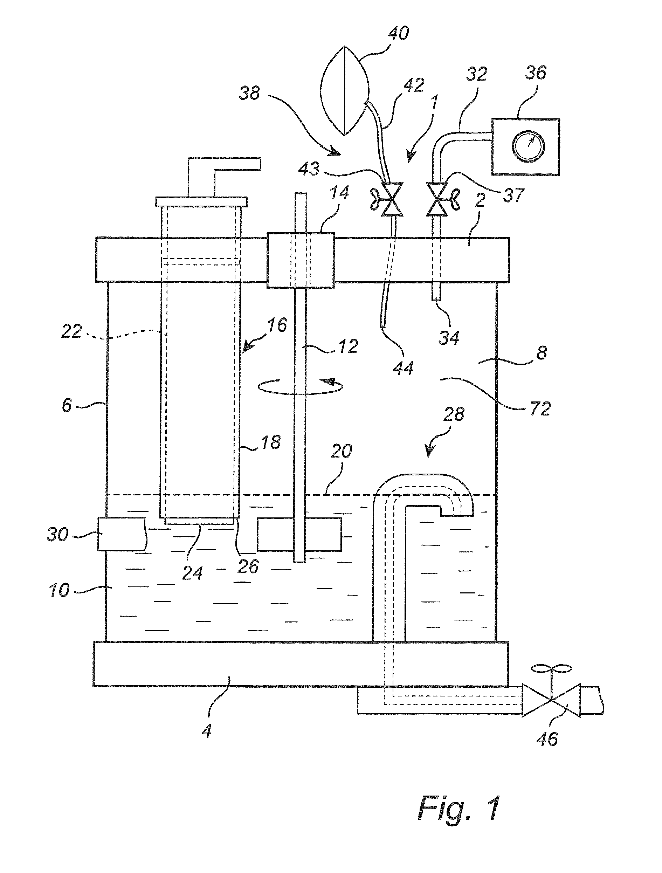

Fig. 1 illustrates a biogas producing laboratory reactor 1. The biogas, which may typically contain methane gas, $CH_4$, carbon dioxide gas, $CO_2$, and, possibly, hydrogen gas, $H_2$, and trace amounts of other gases, such as hydrogen sulphide, $H_2S$, is generated by digestion of organic material under anaerobic conditions using living microorganisms, such as biogas producing bacteria and archaea, in the laboratory reactor 1. The organic material could comprise, for example, various types of materials of biological origin, hereinafter referred to as "biological material", such as slaughterhouse waste, sewage sludge, food waste, agricultural by-products, animal waste products, cow dung, and various mixtures of these and other biological materials. The organic material could also comprise, for example, various types of materials of non-biological origin, hereinafter referred to as "non-biological material", such as residues and waste of paint, ink, and dye, used oil, etc. Furthermore, biogas could be formed by digesting mixtures of biological and non-biological materials. The embodiments described hereinafter mention, as an example, digestion of biological material. Experiments with digestion under anaerobic conditions in the biogas producing laboratory reactor 1 may be performed to find optimum process conditions for, e.g., a particular biological material, or mixture of two or more different organic materials. Once optimum process conditions have been established by the experiments, those process conditions could be applied in a full scale biogas producing plant utilizing a similar type of material.

The biogas producing laboratory reactor 1 comprises a top plate 2, a bottom plate 4 and a cylindrical wall element 6 arranged therebetween. The top plate 2, the bottom plate 4 and the cylindrical wall element 6 form a gas-tight reactor cavity 8 in which biological material 10 of liquid or slurry consistency may be contained. The volume of the gas-tight reactor cavity 8 is typically 1-100 litres, preferably 5-25 litres. Typically, the top and bottom plates 2, 4 may be made from a metal material, such as steel or aluminium, or from a polymer material, such as polypropylene (PP) or polytetrafluoroethylene (PTFE). The cylindrical wall element 6 may be made from one of the above mentioned metal and polymer materials. According to a preferred embodiment the cylindrical wall element 6 is made from a transparent material, such as glass or plastic, such as Duran™ glass.

An optional agitator 12 may extend through a bushing 14 arranged in the top plate 2 for agitating the biological material 10 contained in the reactor cavity 8.

A feeding device 16 is arranged for feeding biological material that is to be digested to the reactor cavity 8 with a minimum of unwanted feed of air to the reactor cavity 8. Oxygen gas comprised in the air is toxic to some biogas producing microorganisms. Furthermore, oxygen comprised in an unwanted feed of air to the reactor cavity 8 may also react with certain gas components, such as hydrogen sulphide, produced in the digestion process, such reaction resulting in incorrect measurement results for such gas components. Still further, an unwanted feed of air to the reactor cavity 8 also dilutes the biogas produced in an unpredictable manner, resulting in uncertainties in the analysis of the amount and composition of the biogas formed. Hence, an efficient device for feeding material to the reactor cavity 8, with minimum supply of air, increases the relevance and reproducibility of the experimental results obtained. The feeding device 16 comprises a feeding tube 18 which is mounted in the top plate 2 and which extends below a liquid surface 20 of the liquid or slurry material 10 contained in the reactor cavity 8. The feeding device 16 further comprises a plunger 22 which is insertable in the feeding tube 18 for pressing down biological material below the liquid surface 20, and for sealing the feeding tube 18, as will be described in more detail hereinafter. A lower end 24 of the plunger 22 extends below a lower end 26 of the feeding tube 18, to ensure that biological material to be fed to the cavity 8 is mixed with the bulk of biological material 10 present inside the cavity 8, and is not stuck inside of the feeding tube 18. The lower ends 24, 26 are arranged for being immersed in the liquid or slurry material 10 during operation of the reactor 1, i.e., the lower ends 24, 26 are located below the surface 20.

A level setting device 28, which will be described in more detail hereinafter, is arranged for adjusting the amount of biological material 10 that is present inside of the cavity 8.

A heating jacket 30, only illustrated in part in Fig. 1, may be swept around the cylindrical wall element 6 to heat the biological material 10 present inside the cavity 8 to a suitable and constant temperature of, typically, 5 to 95°C, more often a temperature of 20-60°C.

Biogas produced inside the cavity 8 is collected via gas pipe 32 extending through the top plate 2. As can be seen from Fig. 1, a lower open end 34 of the gas pipe 32 is arranged well above the liquid surface 20 to avoid contamination of the biogas by liquid material 10. The gas pipe 32 is connected to an analysing instrument 36 which analyses the amount and composition of the biogas produced. An optional valve 37 may be arranged in the gas pipe 32 for shutting off the flow of biogas to the analysing instrument 36 upon feeding or removing material from the cavity 8.

A gas buffering system 38 is arranged for avoiding over and under pressure inside the reactor cavity 8 upon feeding biological material to the cavity 8, or removing digested material from the reactor cavity 8. The gas buffering system 38 comprises an expandable gas bag 40, which is arranged outside of the cavity 8, and a gas pipe 42 fluidly connected to the gas bag 40 and extending through the top plate 2 into the cavity 8 and having a lower open end 44 which is arranged well above the liquid surface 20. The expandable gas bag 40 may contain, due to its expandable characteristics, varying amounts of biogas. An optional valve 43 may be arranged in the gas pipe 42 for opening a gas connection between the cavity 8 and the gas bag 40 upon feeding or removing material from the cavity 8. Hence, as will be described in more detail hereinafter, when biological material is supplied to the cavity 8, thereby increasing the volume of liquid or slurry material 10 inside of the cavity 8, excess biogas is transported to the expandable gas bag 40, such that overpressure inside the cavity 8 is avoided. When digested biological material is removed from the cavity 8, thereby reducing the volume of liquid or slurry material 10 present inside of the cavity 8, biogas stored inside the expandable gas bag 40 is transported to the cavity 8, such that under pressure inside the cavity 8 can be avoided. The latter is beneficial, since under pressure could cause ambient air to be drawn into the cavity 8, which could result in partial poisoning of the biogas producing microorganisms.

Digested biological material may be transported from the cavity 8 via the above mentioned level setting device 28, by opening a valve 46 arranged thereon, outside of the cavity 8.

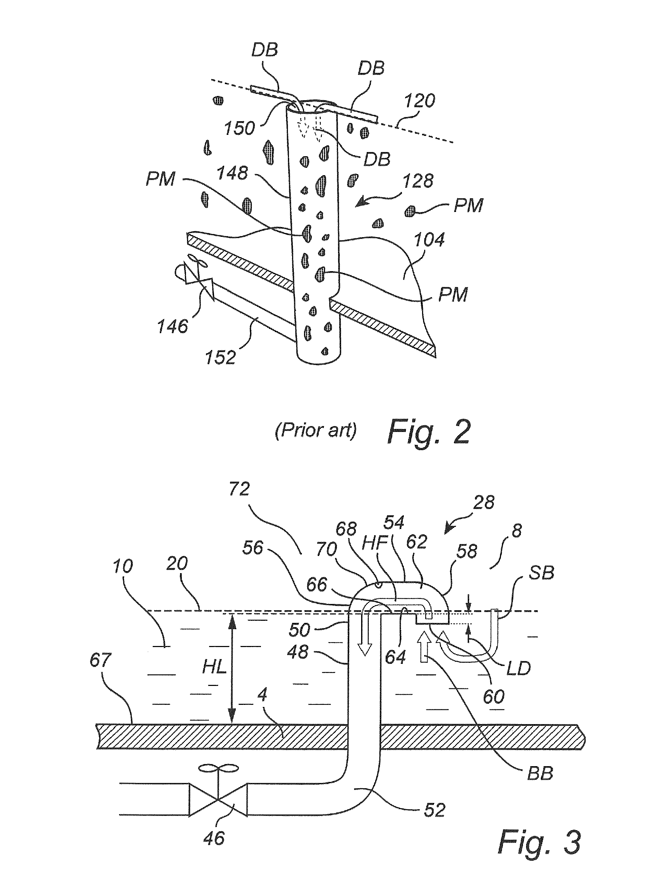

Fig. 2 illustrates a level setting device 128 according to a prior art design. The prior art level setting device 128 comprises a vertical drain pipe 148 which is mounted in a bottom plate 104. The drain pipe 148 has an open upper end 150. The open upper end 150 receives digested biological material at a liquid surface 120. Upon opening a valve 146 digested biological material drain into the open end 150, vertically downwards through the drain pipe 148 and out of the laboratory reactor via a pipe 152 on which the valve 146 is arranged. Arrows DB illustrate how digested biological material at the surface 120 enter the upper open end 150 of the drain pipe 148 and flows downwardly through pipe 148. It has now been found that the open upper end 150 may perform the unwanted function of being a particle trap, with particulate material PM, and in particular the largest particles, being trapped inside of the drain pipe 148, and not being able to escape therefrom. The effect is, as is indicated in Fig. 2, that the concentration of particulate material PM becomes much higher inside of the drain pipe 148, than outside of the drain pipe 148. Upon draining material via the drain pipe 148 the trapped particulate material PM will also be drained, causing a depletion of particulate material PM.

Fig. 3 illustrates the level setting device 28 according to one embodiment of the present invention. The level setting device 28 comprises a vertical drain pipe 48 which is mounted in the bottom plate 4 and which extends vertically upwards from the bottom plate 4. The vertical drain pipe 48 is, at an upper end 50, fluidly connected to a tube bend 54. In the embodiment illustrated in Fig. 3, the tube bend 54 is a 180° bend. The bend 54 is, at a first end 56, fluidly connected to the upper end 50 of the vertical drain pipe 48. At a second end 58, the bend 54 has a drain opening 60 which faces vertically downwards into the cavity 8. The drain opening 60 is adapted to receive digested biological material 10 contained in the cavity 8 of the biogas producing laboratory reactor 1, illustrated in Fig. 1.

Returning to Fig. 3, the bend 54 has an internal bend cavity 62 that provides for a fluid connection between the cavity 8 and the vertical drain pipe 48. The internal bend cavity 62 has a lower flow restriction 64, which, in the embodiment illustrated in Fig. 3, coincides with the inner bottom 66 of the bend 54. The lower flow restriction 64 is located at an intended liquid height HL above an inner bottom 67 of the cavity 8, i.e., at a height HL above the upper surface of the bottom plate 4. The height HL is typically 50 to 1000 mm. The biological material 10 must flow over the lower flow restriction 64 when passing from the drain opening 60 and into the drain pipe 48. The lower flow restriction 64 sets, when operating the device 28, the vertical level of the surface 20 of the material 10 contained in the cavity 8. The lower flow restriction 64 is located in a position which is a vertical distance LD above the position of the drain opening 60. Typically, the vertical distance LD is 5-300 mm, more preferably 8-50 mm, and most preferably 10-40 mm. The vertical distance LD may typically be 1-40 %, more preferably 5-30%, and most preferably 10-20 % of the height HL. Thus, for example, if the height HL is 200 mm, then the vertical distance LD could suitably be 15 % of that value, namely 30 mm.

When the volume of material 10 inside of the cavity 8 is to be adjusted, the valve 46 is opened. Upon opening the valve 46 digested biological material flows into the drain opening 60, further through the internal bend cavity 62 of the bend 54, as indicated by an arrow HF, and further to the drain pipe 48. The material then flows vertically downwards through the drain pipe 48 and out of the laboratory reactor via a pipe 52 on which the valve 46 is arranged. Hence, the material may be drained out of the cavity 8 under the force of gravity. Optionally, the pipe 52 may be connected to a water trap (not shown) to further reduce the risk that air enters the cavity 8. As illustrated in Fig. 3, material 10 enters the drain opening 60 both from adjacent the liquid surface 20, as indicated by an arrow SB, and from the bulk of the material 10, as indicated by an arrow BB. Hence, the material 10 entering the drain opening 60 and being drained from the cavity 8, via the bend 54 and the pipe 48, will be a representative average of the material present in the cavity 8. Thus, the solid retention time, SRT, i.e., the number of days that solid material is retained inside of the cavity 8 before being drained therefrom, will be the same for large and small particles. In situations of microorganisms, such as biogas producing bacteria and archaea, growing on particles, the retention time of such microorganisms inside of the cavity 8 will be representative regardless of whether such microorganisms tend to grow on large or small particles. Material 10 drains out of the cavity 8 until the surface 20 reaches the same vertical level as the lower flow restriction 64. Hence, the lower flow restriction 64 sets a vertical level of material 10 inside of the cavity 8. When the surface 20 reaches the vertical level of the flow restriction 64, the drainage of material out of the cavity 8 will immediately cease, and the surface 20 will remain at the vertical level of the lower flow restriction 64 until more material to be digested is fed to the cavity 8. The valve 46 may be closed to avoid that air enters the cavity 8 via the vertical drain pipe 48.

When the surface 20 reaches the vertical level of the flow restriction 64, and drainage of material 10 via the level setting device 28 stops, the drain opening 60 is still immersed in the material, below the surface 20, since the flow restriction 64 is located in a position which is a vertical distance LD above the position of the drain opening 60. Hence, the drain opening 60 does not collect material from the surface, as may be the case in the prior art, illustrated in Fig. 2.

When material stops flowing out of the cavity 8 via the pipe 52 the valve 46 is closed. Hence, the level setting device 28 makes it possible to keep, in a simple manner, a very exact amount of material 10 inside of the cavity 8, and to drain a representative average of the material 10 contained in the cavity 8.

In accordance with an alternative embodiment the pipe 52 could be provided with a water seal, such that material could be drained from the cavity 8 without having to control a valve 46.

The bend 54 may, optionally, be provided with a ventilation opening 68. The ventilation opening 68 is formed in an upper portion 70 of the bend 54. The ventilation opening 68 fluidly connects the interior of the level setting device 28 to a biogas reactor headspace 72 of the cavity 8 for equalizing the gas pressure between the interior of the level setting device 28 and the cavity 8. By means of the ventilation opening 68, the risk of under pressure being generated inside of the bend 54 is reduced. Thereby, the risk of unwanted siphoning effects in the level setting device 28 is reduced.

An experiment was conducted to compare the level setting device 28 described with reference to Fig. 3 to the prior art level setting device 128 described in Fig. 2. A laboratory reactor digested biological material to produce biogas. The biological material comprised about 50% slaughterhouse waste, and about 50% general food waste. In Comparative test A and Comparative test B the volume of the reactor was adjusted by means of the prior art level setting device 128.

In Test 1, 2 and 3 the volume of the reactor was adjusted by means of the level setting device 28. In the tests, the dry solids content of the material being drained from the reactor via the respective pipe 152, 52 was compared to the global dry solids content, measured by direct sampling from the cavity 8 of the laboratory reactor.

The dry solids contents of the samples were measured according to Swedish standard SS 02 81 13. According to Swedish standard SS 02 81 13 material is to be weighed and is then to be heat treated at 105°C for 20 h to evaporate water. The material is then to be weighed once more. The dry solids content in % by weight DS is then calculated as follows:

$$\% \text{ by weight DS} = \frac{\text{weight after heating at } 105^{\circ}\text{C}}{\text{weight before heating}} * 100\%$$

For instance, 6% by weight DS relates to a material where 6% of the original weight of the material remains after heating the material at 105°C for 20 h.

The following dry solids contents were measured in the experiment:

Table 1: Comparison of DS of drained sample

| Test | Drained sample [% weight DS] | Global sample [% weight DS] | Difference [% weight DS] |
|---|---|---|---|
| Comparative A | 7.9 | 6.0 | 1.9 |
| Comparative B | 6.8 | 6.0 | 0.8 |
| Avg. Comparative | 7.35 | 6.0 | 1.35 |
| Test 1 | 6.7 | 6.0 | 0.7 |
| Test 2 | 5.2 | 5.9 | -0.7 |
| Test 3 | 5.5 | 5.8 | -0.3 |
| Avg. Tests | 5.8 | 5.9 | -0.1 |

As can be seen from Table 1, the average difference in the Comparative tests with the prior art level setting device 128 results in an average deviation in the dry solids content of 1.35% units, when comparing the dry solids content of the drained material to the dry solids content of the bulk of material in the reactor. The tests with the level setting device 28 results, on the other hand, in an average deviation in the dry solids content of only 0.1 % units, when comparing the dry solids content of the drained material to the dry solids content of the bulk of material in the reactor. Hence, with the level setting device 28 it is possible to drain a material from the reactor which is truly representative of the bulk of material present in the reactor. It will be appreciated that with the prior art, the dry solids content, % by weight DS, of the bulk material inside of the reactor may, after some time of operation, be reduced in a manner which is not caused by the digestion process itself, but by the manner of draining material. This reduces the accuracy and relevance of test results obtained. Furthermore, in the prior art the degree of degradation may be incorrectly measured, since some large particles may be drained long before they have had a chance to become properly digested.

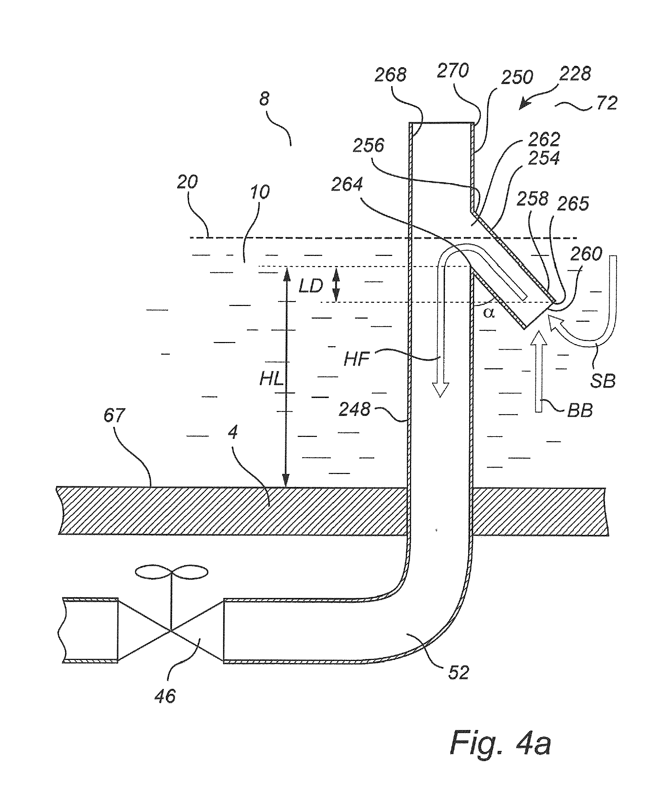

Fig. 4a illustrates a level setting device 228 according to a second embodiment. The level setting device 228 comprises a vertical drain pipe 248 which is mounted in the bottom plate 4 and which extends vertically upwards from the bottom plate 4. The vertical drain pipe 248 is, at an upper end 250, fluidly connected to a branch tube 254. The branch tube 254 extends, at an angle α of about 45°, downwards from the upper end 250 of the vertical drain pipe 248 and into the material 10 of the cavity 8. Typically, the angle α would be in the range of 10 to 75°. The branch tube 254 is, hence, at a first end 256, fluidly connected to the upper end 250 of the vertical drain pipe 248. At a second end 258, the branch tube 254 has a drain opening 260 which faces downwards into the cavity 8. The branch tube 254 has an internal tube cavity 262 that provides for a fluid connection between the cavity 8 and the vertical drain pipe 248. At the connection between the branch tube 254 and the vertical drain pipe 248 a lower flow restriction 264 is formed. The lower flow restriction 264 sets, when operating the device 228, the vertical level of the surface 20 of the material 10 contained in the cavity 8. The lower flow restriction 264 is located at an intended liquid height HL of typically 50 to 1000 mm above the inner bottom 67 of the cavity 8. The lower flow restriction 264 is located in a position which is a vertical distance LD above an upper end 265 of the opening 260. Typically, the vertical distance LD is 5-300 mm, more preferably 8-50 mm, and most preferably 10-40 mm. The vertical distance LD may typically be 1-40 %, more preferably 5-30%, and most preferably 10-20 % of the height HL.

Adjusting the volume of material 10 inside of the cavity 8 can be initiated in a similar manner as with the level setting device 28 described hereinbefore, by opening the valve 46. Upon opening the valve 46 digested biological material flows into the drain opening 260, further through the internal tube cavity 262 of the branch tube 254 and into the drain pipe 248, as indicated by an arrow HF. The material then flows out of the laboratory reactor via the pipe 52 on which the valve 46 is arranged. Material 10 enters the drain opening 260 both from adjacent the liquid surface 20, as indicated by an arrow SB, and from the bulk of the material 10, as indicated by an arrow BB.

Material 10 drains out of the cavity 8 until the surface 20 reaches the same vertical level as the lower flow restriction 264. When the surface 20 reaches the vertical level of the flow restriction 264, the drainage of material out of the cavity 8 will immediately cease, and the surface 20 will remain at the vertical level of the lower flow restriction 264 until more material to be digested is fed to the cavity 8.

The vertical drain pipe 248 may, optionally, be provided with a ventilation opening 268. The ventilation opening 268 is formed as an upper open end portion 270 of the pipe 248 and is in contact with biogas reactor headspace 72. By means of the ventilation opening 268, the risk of under pressure being generated inside of the level setting device 228 is reduced, such that the risk of unwanted siphoning effects in the level setting device 228 is reduced. If the vertical level of the surface 20 varies within a wide range, the pipe 248 can be extended vertically upwards to accommodate for such variations, to avoid that material enters the drain pipe 248 via the ventilation opening 268.

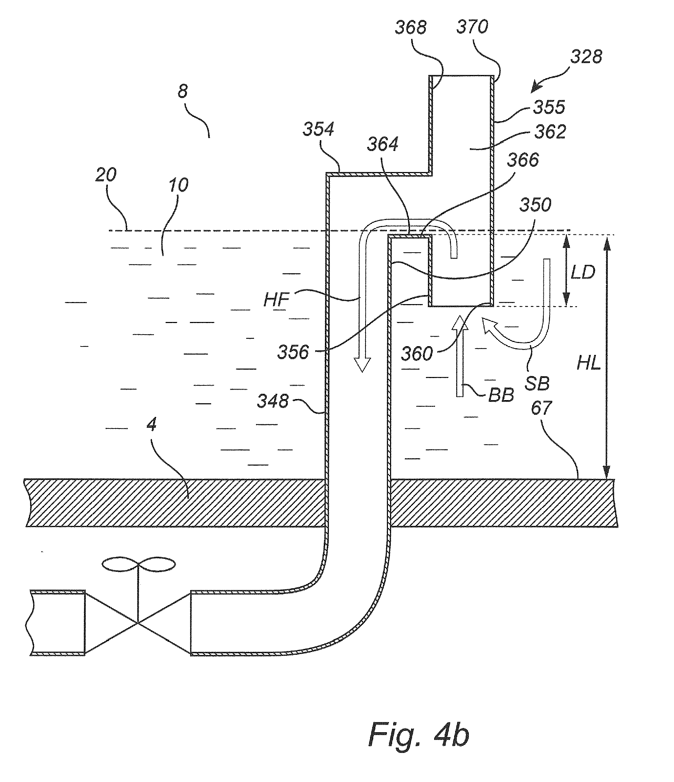

Fig. 4b illustrates a level setting device 328 according to a third embodiment. The level setting device 328 comprises a vertical drain pipe 348 which extends vertically upwards from the bottom plate 4. The vertical drain pipe 348 is, at an upper end 350, fluidly connected to an elbow pipe 354. The elbow pipe 354 is fluidly connected to a vertical combined opening and ventilation pipe 355. The vertical combined opening and ventilation pipe 355 has, at a lower first end 356, a drain opening 360 which faces downwards into the cavity 8. The pipe 355 has an internal tube cavity 362 that provides for a fluid connection between the cavity 8 and the vertical drain pipe 348, via the elbow pipe 354. An inner bottom 366 of the elbow pipe 354 forms a lower flow restriction 364 which sets, when operating the device 328, the vertical level of the surface 20 of the material 10 contained in the cavity 8. The lower flow restriction 364 is located at an intended liquid height HL of typically 50 to 1000 mm above the inner bottom 67 of the cavity 8. The lower flow restriction 364 is located in a position which is a vertical distance LD of 5-300 mm, more preferably 8-50 mm, and most preferably 10-40 mm, above the opening 360. The vertical distance LD may typically be 1-40 %, more preferably 5-30%, and most preferably 10-20 % of the height HL.

Adjusting the volume of material 10 inside of the cavity 8 by draining material 10 into the opening 360 of the pipe 355, indicated by arrows SB and BB, and out of the cavity 8, indicated by arrow HF, until the surface 20 reaches the same vertical level as the lower flow restriction 364 is performed in accordance with similar principles as described hereinbefore with reference to Fig. 4a.

The vertical combined opening and ventilation pipe 355 may, optionally, be provided with a ventilation opening 368. The ventilation opening 368 is formed as an upper open end portion 370 of the pipe 355. By means of the ventilation opening 368, the risk of under pressure being generated inside of the level setting device 328 is reduced, such that the risk of unwanted siphoning effects in the level setting device 328 is reduced. The pipe 355 can be extended vertically upwards to accommodate for large variations in the level 20 of material 10 in the cavity 8.

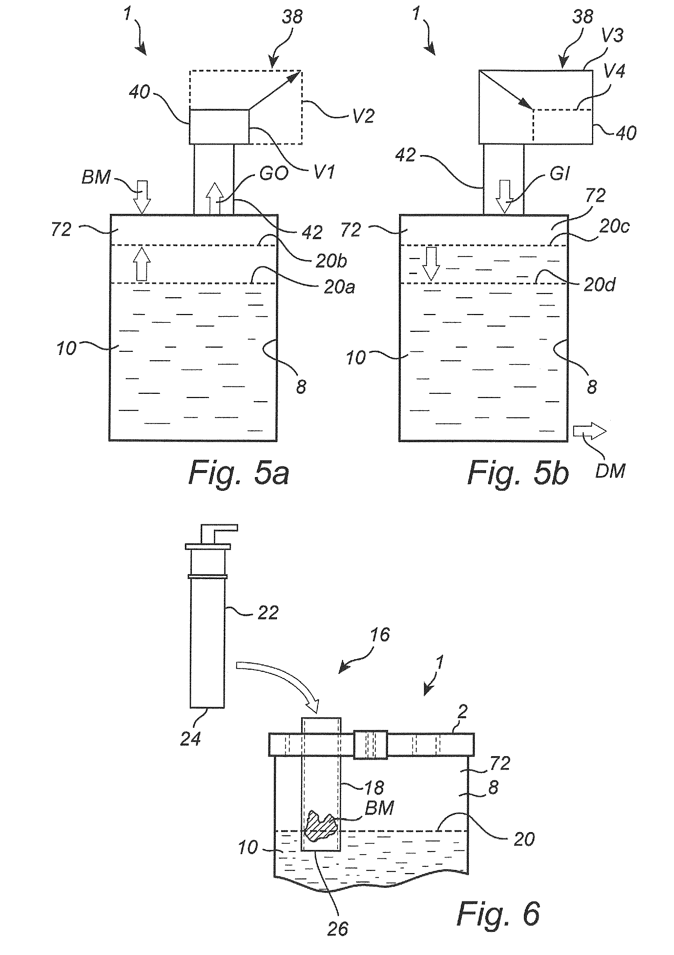

Fig. 5a is a schematic side view of the gas buffering system 38 in a first mode of operation. In this first mode of operation biological material BM to be digested is supplied to the cavity 8 of the biogas producing laboratory reactor 1. According to one embodiment the supply of material to the cavity 8 is preceded by shutting optional valve 37, illustrated in Fig. 1, and opening optional valve 43, also illustrated in Fig. 1, such that gas bag 40 becomes fluidly connected to the cavity 8, and in particular to the biogas reactor headspace 72. Hence, changes in gas volume during material feeding will only affect the gas bag 40 of the gas buffering system 38, and not the analysing instrument 36. Then, supply of material BM to the cavity 8 may commence. As an effect of such supply, the volume of material 10 inside of the cavity 8 increases, as illustrated by means of an arrow, causing a surface of the material 10 to rise from a first surface level 20a to a second, higher, surface level 20b. The rise in the surface level from first level 20a to second level 20b causes a corresponding reduction in volume of the biogas reactor headspace 72 of the cavity 8. As an effect of such reduction in the volume of the biogas reactor headspace 72, biogas GO is pressed out of the cavity 8, via the gas pipe 42 and into the expandable gas bag 40. As an effect of such amount of gas GO entering the gas bag 40 the gas bag expands to an increased volume, as indicated with dashed lines in Fig. 5a, from a first volume V1 to a second volume V2. The available volume of the gas bag 40 is considerably larger than the amount of gas GO entering the gas bag 40, meaning that no substantial pressure is built up inside the gas bag 40. In one example the gas bag 40 is a FlexFoil™ Sample Bag with a volume of 3 litres, as available from SKC Inc, PA, USA. In one example, the amount of gas GO would represent a volume, at atmospheric pressure, of 10 to 500 ml of biogas, and the second volume V2 would represent about 2000 ml of biogas, at atmospheric pressure. The pressure inside the gas bag 40 will, after receiving the gas GO, remain at close to atmospheric pressure. The fact that the expandable gas bag 40 expands upon receiving the gas GO means that no substantial increase in the pressure in the gas bag 40 or in the biogas reactor headspace 72 of the cavity 8 occurs upon supplying biological material BM. Hence, the digestion of the material 10 inside of the cavity 8 may continue at close to atmospheric pressure conditions also after the supply of the biological material BM. The supply of material BM to the cavity 8 is succeeded by opening optional valve 37 and shutting optional valve 43, such that analysing instrument 36 becomes again fluidly connected to biogas reactor headspace 72.

Fig. 5b is a schematic side view of the gas buffering system 38 in a second mode of operation. In the second mode of operation digested biological material DM is drained from the cavity 8 of the biogas producing laboratory reactor 1. Draining digested material DM could be performed using, for example, the level setting device 28 described hereinbefore with reference to Fig. 3. According to one embodiment the draining of material from the cavity 8 is preceded by shutting optional valve 37 of Fig. 1, and opening optional valve 43 of Fig. 1, such that gas bag 40 becomes fluidly connected to the biogas reactor headspace 72. Hence, changes in gas volume during draining of material will only affect the gas bag 40 of the gas buffering system 38, and not the analysing instrument 36. Then, draining of material from the cavity 8 may commence. As an effect of such draining of material DM, the volume of material 10 inside of the cavity 8 decreases, as illustrated by means of an arrow, causing a surface of the material 10 to fall from a third surface level 20c to a fourth, lower, surface level 20d. The fall in the surface level from third level 20c to fourth level 20d causes a corresponding expansion in volume of the biogas reactor headspace 72 of the cavity 8. As an effect of such expansion of the volume of the biogas reactor headspace 72, gas GI is drawn into the cavity 8 from the expandable gas bag 40, via the gas pipe 42. As an effect of such amount of gas GI leaving the gas bag 40 the gas bag is compressed, under the atmospheric pressure surrounding the gas bag 40, to a reduced volume, as indicated with dashed lines in Fig. 5b, from a third volume V3 to a fourth volume V4. The volume of biogas originally available in the gas bag 40 is considerably larger than the amount of gas GI being drawn out of the gas bag 40. In one example, the third volume V3 would represent about 2000 ml of biogas, with the amount of gas GI representing a volume, at atmospheric pressure, of 10 to 500 ml of biogas. Hence, the fourth volume V4 would be in the range of 1500 to 1990 ml of biogas. The pressure inside the gas bag 40 will, after losing the amount of gas GI, remain at close to atmospheric pressure. The fact that biogas is transferred from the expandable gas bag 40, as the gas GI, into the biogas reactor headspace 72 of the cavity 8 means that no under pressure occurs in the biogas reactor headspace 72 of the cavity 8 upon draining digested biological material DM. Hence, the digestion of the material 10 inside of the cavity 8 may continue at close to atmospheric pressure conditions also after the draining of the digested biological material DM. Furthermore, the gas GI being transferred from the gas bag 40 to the biogas reactor headspace 72 of the cavity 8 is biogas having substantially the same properties as the biogas already present inside the biogas reactor headspace 72. This means that the gas conditions both as regards gas pressure and gas composition inside of the biogas reactor headspace 72 of the cavity 8 will remain the same also after material DM has been drained from the cavity 8. The draining of material from the cavity 8 is succeeded by opening optional valve 37 and shutting optional valve 43, such that analysing instrument 36 becomes again fluidly connected to biogas reactor headspace 72.

With the gas buffering system 38 described with reference to Figs. 5a and Fig. 5b it becomes possible to operate the biogas producing laboratory reactor 1 at constant, or close to constant, pressure conditions. Furthermore, the risk is reduced that biogas leaks out of the cavity 8 as an effect of any overpressure generated when supplying biological material BM to the reactor 1. Still further, the risk is reduced that air leaks into the cavity 8 as an effect of any under pressure generated when draining biological material DM from the reactor 1. The latter is beneficial since the oxygen contained in the air may be harmful to the biogas producing microorganisms. Dilution of the biogas and/or reactions between oxygen and gas components, such as hydrogen sulphide, of the biogas may also distort the test results. At start-up of the biogas producing laboratory reactor 1 a portion of the biogas produced may be directed to the gas bag 40, instead of via the gas pipe 32 and the analysing instrument 36, to fill the gas bag 40 up to about 1/2 to 2/3 of its total volume with biogas, which is then used as a buffer to compensate for biological material being supplied to or drained from the cavity 8, in accordance with the above description. According to an alternative embodiment the gas buffering system 38 is a passive system, meaning that biogas can freely flow between the gas bag 40 and the biogas reactor headspace 72, via the gas pipe 42, as biological material is supplied to or removed from the cavity 8, with no need for controlling the flow of biogas via pipe 42 by valves or similar devices.

Fig. 6 is a schematic side view of the feeding device 16 of the biogas producing laboratory reactor 1 in a first stage of feeding biological material BM to the cavity 8. When feeding biological material to the cavity 8, the plunger 22 of the feeding device 16 is firstly removed from the feeding tube 18 extending through the top plate 2. As can be seen from Fig. 6, the feeding tube 18 extends below the liquid surface 20, which means that there is no contact between the biogas reactor headspace 72 of the cavity 8 and the atmosphere even when the plunger 22 has been removed. Hence, biogas does not leak out of the cavity 8, and air does not enter the cavity 8 when the plunger 22 has been removed from the feeding tube 18. A lump of biological material BM has been dropped down inside feeding tube 18 and floats on the liquid surface 20 of the biological material 10. The plunger 22 is then, as illustrated by an arrow, inserted in the feeding tube 18 and forced downwards to press biological material BM into the bulk of material 10. As described hereinbefore with reference to Fig. 1, the lower end 24 of the plunger 22 extends below the lower end 26 of the feeding tube 18, which means that the biological material BM cannot remain inside of the feeding tube 18, but is pushed out of the feeding tube 18 by plunger 22 lower end 24 and into the bulk of material 10.

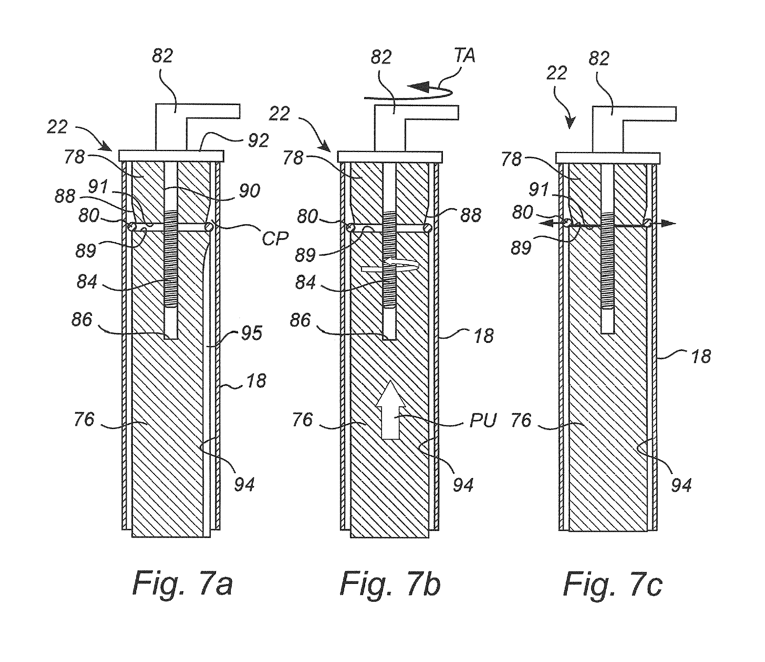

Fig. 7a illustrates the plunger 22 in more detail just after it has been inserted in feeding tube 18. The plunger 22 comprises, as its main parts, a lower plunger portion 76, an upper plunger portion 78, a sealing ring 80, which may be made of rubber or other suitable material, a manoeuvring handle 82, and a threaded bar 84. The lower plunger portion 76 comprises a vertical threaded hole 86 which may receive the threaded bar 84. The upper plunger portion 78 comprises a lower conical portion 88 which tapers in a downward direction and around which the sealing ring 80 is mounted. The sealing ring 80 rests on an upper surface 89 of the lower plunger portion 76. The upper plunger portion 78 also comprises a vertical central hole 90 through which the threaded bar 84 extends. The manoeuvring handle 82 is rigidly connected to the threaded bar 84 and abuts an upper portion 92 of upper plunger portion 78. In the moment illustrated in Fig. 7a, the plunger 22 has just been inserted into the feeding tube 18, but no sealing action has been initiated. Hence, there is a clearance CP between the sealing ring 80 and an inner wall 94 of the feeding tube 18. This clearance CP makes it possible for air present inside of feeding tube 18 to escape upwards when the plunger 22 is gradually moved vertically downward inside of tube 18, such that the air is not pushed into the material 10 inside the cavity 8. Optionally, the lower plunger portion 76 may be provided with a notch 95 extending from a lower end of the lower plunger portion 76 and up to a position just below the sealing ring 80. The notch 95, which may typically have a cross-section of 2-4 mm by 2-4 mm, makes it even easier for air present inside of feeding tube 18 to escape upwards when the plunger 22 is gradually moved vertically downward inside of tube 18.

Fig. 7b illustrates the tightening of the plunger 22 to the feeding tube 18. The manoeuvring handle 82 is turned in a clock-wise direction, as illustrated by an arrow TA. Turning the handle 82 makes the threaded bar 84 turn inside of the threaded hole 86 of the lower plunger portion 76. The threaded bar 84 thereby pulls the lower plunger portion 76 upwards, as illustrated by an arrow PU. With the lower plunger portion 76 being pulled upwards, the upper surface 89 thereof presses the sealing ring 80 upwards on the lower conical portion 88. Such pressing causes the sealing ring 80 to expand in the horizontal direction, such that the clearance CP is closed, and the sealing ring 80 forms a seal between the plunger 22 and the inner wall 94 of the feeding tube 18.

Fig. 7c illustrates the plunger 22 when in its sealing position. In such position the sealing ring 80 seals the upper plunger portion 78 and the lower plunger portion 76 to the inner wall 94 of the feeding tube 18. A sealed position may coincide with the upper surface 89 of the lower plunger portion 76 abutting a lower surface 91 of the upper plunger portion 78. When new material is to be fed to the cavity 8 of the biogas producing laboratory reactor 1 the handle 82 is turned in the counter-clockwise direction to lower the lower plunger portion 76 and to release the sealing function of sealing ring 80, after which the plunger 22 may be lifted out of the feeding tube 18.

By means of the feeding device 16 it becomes possible to feed biological material BM to the cavity 8 of the biogas producing laboratory reactor 1 in such manner that it is ensured that the biological material BM mixes well with the bulk of material 10 already present inside the cavity 8 of the reactor 1. Furthermore, leakage of biogas out of the cavity 8 is prevented, both when removing the plunger 22 from the feeding tube 18, and when inserting the plunger 22 into the feeding tube 18. Still further, leakage of air into the cavity 8, and associated contamination of biogas producing microorganisms by oxygen, is prevented, both when removing the plunger 22 from the feeding tube 18, and when inserting the plunger 22 into the feeding tube 18.

Further solutions:

According to one alternative solution for obtaining reliable small scale experiments with digestion a laboratory reactor comprises a cavity for containing organic material during the digestion thereof, wherein the laboratory reactor may further comprise a gas buffering system having an expandable gas bag and a gas pipe connecting the gas bag to a biogas reactor headspace of the cavity, the gas buffering system being adapted to transport biogas contained in the expandable gas bag to the biogas reactor headspace of the cavity when adjusting the volume of organic material contained in the cavity by means of a level setting device. Such a laboratory reactor having a gas buffering system makes it possible to avoid over and under pressure inside the reactor cavity upon feeding biological material to the cavity, or removing digested material from the reactor cavity. The gas buffering system of this alternative solution may be used independently for improving the control of the process occurring in a laboratory reactor, or may be used in a laboratory reactor together with the level setting device and/or the feeding device described hereinbefore for further improved control of small scale experiments with digestion of organic material.

According to a still further alternative solution for obtaining reliable small scale experiments with digestion a laboratory reactor may comprise a cavity for containing organic material during the digestion thereof, wherein the laboratory reactor further comprises a feeding device comprising a feeding tube adapted to extend below a liquid surface of the organic material contained in the cavity. Thereby, newly fed material may be quickly immersed into and mixed with the bulk of material in the cavity, and can be accessible for digestion almost immediately after being fed to the cavity. According to one embodiment the feeding device comprises a plunger for forcing organic material fed to the feeding tube into the bulk of organic material. Thereby, material may be more efficiently forced into the bulk of material in the cavity, to obtain quick mixing. According to one embodiment, the plunger of the feeding device may be provided with a notch extending along the length of the plunger, to enable air of the feeding tube to escape when the plunger is utilized for supplying material to the bulk of organic material in the cavity. Thereby, the risk that air is inadvertently injected into the cavity while injecting material to be digested is reduced. The feeding device of this alternative solution may be used independently for improving the control of the process occurring in a laboratory reactor, or may be used in a laboratory reactor together with the level setting device and/or the gas buffering system described hereinbefore for further improved control of small scale experiments with digestion of organic material.

Alternative embodiments:

It will be appreciated that numerous variants of the embodiments described above are possible within the scope of the appended claims.

Hereinbefore it has been described that the drain opening may either be arranged facing vertically downwards, as is the case with the drain opening 60 illustrated in Fig. 3, and the drain opening 360 illustrated in Fig. 4b, or facing downwards in an inclined manner as is the case with the drain opening 260 illustrated in Fig. 4a. It will be appreciated that it is also possible to have the drain opening facing horizontally, or even upwards, as long as the entire opening is located below the level of the lower flow restriction 64, 264, 364 respectively. It is often preferred that the drain opening faces downwards, either vertically downwards, or in an inclined manner, since this normally yields minimum flow restrictions for material flowing through the level setting device.

Hereinbefore it has been described that the drain pipe 48, 248, 348 is a straight vertical pipe extending through the bottom plate 4 of the reactor 1. It will be appreciated that the drain pipe could also be an inclined, and/or bent pipe, and that the drain pipe could extend through the bottom plate 4 or through the wall element 6.

Hereinbefore it has been described that the laboratory reactor 1 is utilized for digestion of organic material. It will be appreciated that the laboratory reactor may also be utilized for experiments with pre-hydrolysis digestion, in which an organic material is partly digested, and is then forwarded to another reactor for further digestion. Furthermore, the laboratory reactor 1 may also be utilized for experiments with dark fermentation in which a material is digested with an aim to generate a biogas that is very rich in hydrogen gas, $H_2$.

Hereinbefore it has been described that valve 37 is shut and valve 43 is opened when material is supplied to or drained from cavity 8 of the reactor 1. It is also possible to arrange the reactor 1 without one or both of these valves, in which case the biogas reactor headspace 72 is always in fluid contact with the gas bag 40 and/or the analysing instrument 36. In some cases, however, it is easier to control the flows of biogas by operating valves 37, 43 in accordance with the above disclosure.

**Claims**

1.    A biogas producing laboratory reactor (1) comprising a cavity (8) for containing organic material during the digestion thereof, and a level setting device (28) comprising a drain pipe (48) via which organic material contained inside of the cavity (8) can be drained out of the cavity (8), the laboratory reactor (1) being **characterised in** the level setting device (28; 228) further comprising:

a drain opening (60; 260) which is fluidly connected to the drain pipe (48; 248) and through which material may be drained from the cavity (8) and into the drain pipe (48: 248), and
a lower flow restriction (64; 264) which sets a vertical level of material inside of the cavity (8) and over which the organic material must flow when passing from the drain opening (60; 260) and into the drain pipe (48: 248), wherein the lower flow restriction (64; 264) is located on a higher vertical level than the drain opening (60; 260).

2.    A laboratory reactor according to claim 1, wherein the lower flow restriction (64; 264) is located at a vertical distance (LD) of 5-300 mm above the position of the drain opening (60; 260).

3.    A laboratory reactor according to any one of the preceding claims, wherein the lower flow restriction (64; 264) is located at an intended liquid height (HL) above an inner bottom (67) of the cavity (8), wherein a vertical distance (LD) between the position of the drain opening (60; 260) and the lower flow restriction (64; 264) amounts to 1-40 % of the intended liquid height (HL).

4.    A laboratory reactor according to any one of the preceding claims, wherein the level setting device (28; 228) further comprises a ventilation opening (68; 268) for fluidly connecting the interior of the level setting device (28; 228) to a gas space (72) for preventing a siphoning effect in the level setting device (28; 228).

**5.** A laboratory reactor according to any one of the preceding claims,
wherein the drain opening (60; 260) faces downwards.

**6.** A laboratory reactor according to any one of the preceding claims,
wherein the drain opening (60) and the drain pipe (48) are fluidly connected to each other by means of a tube bend (54).

**7.** A laboratory reactor according to any one of the preceding claims,
wherein the drain opening (260) and the drain pipe (248) are fluidly connected to each other by means of a branch tube (254).

**8.** A laboratory reactor according to any one of the preceding claims,
wherein the laboratory reactor (1) comprises a gas buffering system (38) having an expandable gas bag (40) and a gas pipe (42) connecting the gas bag (40) to a biogas reactor headspace (72) of the cavity (8), the gas buffering system (38) being adapted to transport biogas contained in the expandable gas bag (40) to the biogas reactor headspace (72) of the cavity (8) when adjusting the volume of organic material contained in the cavity (8) by means of the level setting device (28).

**9.** A laboratory reactor according to any one of the preceding claims,
wherein the total volume of the cavity (8) is 1-100 litres.

**10.** A method of performing a laboratory experiment of digesting organic material in a biogas producing laboratory reactor (1), the method comprising:

feeding organic material (BM) to a cavity (8) of the laboratory reactor (1),
digesting the organic material inside the cavity (8) under anaerobic conditions with production of biogas, and
adjusting the amount of material in the cavity (8) by a level setting device (28) comprising a drain pipe (48; 248) via which material contained inside of the cavity (8) can be drained out of the cavity (8), such material being drained out of the cavity (8) via a drain opening (60; 260) which is fluidly connected to the drain pipe (48; 248) until a vertical level of material inside of the cavity (8) reaches a lower flow restriction (64; 264) which is located on a higher vertical level than the drain opening (60; 260) and over which the organic material must flow when passing from the drain opening (60; 260) and into the drain pipe (48; 248).

**11.** A method according to claim 10, further comprising transporting biogas contained in an expandable gas bag (40) to a biogas reactor headspace (72) of the cavity (8) when adjusting the amount of organic material contained in the cavity (8) by means of the level setting device (28; 228).

**12.** A method according to any one of claims 10-11, further comprising feeding organic material (BM) to be digested to the cavity (8) via a feeding tube (18) extending below a liquid surface (20) of the organic material (10) contained in the cavity (8).

**13.** A method according to claim 12, further comprising utilizing a plunger (22) for forcing organic material (BM) fed to the feeding tube (18) into the bulk of organic material (10).

**14.** A method according to any one of claims 10-13, further comprising transporting biogas contained in a biogas reactor headspace (72) of the cavity (8) to an expandable gas bag (40) when feeding organic material (BM) to the cavity (8).

**15.** A method according to any one of claims 10-14, further comprising ventilating the interior of the level setting device (28; 228) to a gas space (72) for preventing a siphoning effect in the level setting device (28; 228).

EP 2 589 429 A1

Fig. 1

(Prior art)    Fig. 2

Fig. 3

Fig. 4a

Fig. 4b

*Fig. 5a*

*Fig. 5b*

*Fig. 6*

Fig. 7c

Fig. 7b

Fig. 7a

**EUROPEAN SEARCH REPORT**

Europäisches
Patentamt
European
Patent Office
Office européen
des brevets

Application Number

EP 12 18 8375

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 370 418 A (KOOPMAN BENJAMIN L ET AL) 25 January 1983 (1983-01-25) * column 3, line 28 - column 6, line 43 * * figures 2,3A-3C * ----- | 1-15 | INV. C01M41/34 C12M21/107 B01J19/00 B01J19/18 |
| X | EP 0 013 538 A1 (MANAHL ROBERT DIPL ING [AT]) 23 July 1980 (1980-07-23) * page 1, lines 1-10 * * page 9, line 22 - page 10, line 32 * * figures 2,3 * ----- | 1-15 | |
| X | FR 2 477 364 A1 (STEPHANOIS CONSTR MECA [FR]) 11 September 1981 (1981-09-11) * page 1, lines 1-5 * * page 2, line 12 - page 4, line 21 * * page 5, lines 8-23 * * figures 1,3,4 * ----- | 1-15 | |
| A | US 2011/229951 A1 (SPARGO BARRY J [US] ET AL) 22 September 2011 (2011-09-22) * paragraphs [0025] - [0027], [0046] - [0048] * * figure 2 * ----- | 1-15 | TECHNICAL FIELDS SEARCHED (IPC) B01J C12M C01B |
| A | US 4 436 818 A (WIDMER PETER [CH]) 13 March 1984 (1984-03-13) * column 4, line 66 - column 5, line 2 * * column 5, line 63 - column 6, line 66 * * claims 1,2; figure 1 * ----- | 1-15 | |
| A | US 4 401 441 A (CHASE GREGORY J [US]) 30 August 1983 (1983-08-30) * paragraphs [0008] - [0010], [0015], [0016] * * figure 1 * ----- -/-- | 1-15 | |

The present search report has been drawn up for all claims

| Place of search | Date of completion of the search | Examiner |
|---|---|---|
| The Hague | 1 February 2013 | Baumlin, Sébastien |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons

& : member of the same patent family, corresponding
　　document

EPO FORM 1503 03.82 (P04C01)

Europäisches
Patentamt
European
Patent Office
Office européen
des brevets

**EUROPEAN SEARCH REPORT**

Application Number

EP 12 18 8375

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE 20 2009 017017 U1 (NQ ANLAGENTECHNIK GMBH [DE]) 3 February 2011 (2011-02-03) * column 1, lines 5-19 * * column 3, lines 16-20 * * column 6, lines 14-40 * * figure 2 * ----- | 1-15 | |
| A | WO 99/67176 A1 (SUPERGAS A S [DK]; MALLAN JOHANNES JACOBUS [DK]; FENGER JAKOB [DK]; NI) 29 December 1999 (1999-12-29) * the whole document * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 1 February 2013 | Baumlin, Sébastien |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

......................................................................................
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 12 18 8375

This annex lists the patent family members relating to the patent documents cited in the above-mentioned European search report.
The members are as contained in the European Patent Office EDP file on
The European Patent Office is in no way liable for these particulars which are merely given for the purpose of information.

01-02-2013

| Patent document cited in search report | | Publication date | Patent family member(s) | | Publication date |
|---|---|---|---|---|---|
| US 4370418 | A | 25-01-1983 | NONE | | |
| EP 0013538 | A1 | 23-07-1980 | DE | 2965161 D1 | 11-05-1983 |
| | | | EP | 0013538 A1 | 23-07-1980 |
| | | | US | 4302329 A | 24-11-1981 |
| FR 2477364 | A1 | 11-09-1981 | NONE | | |
| US 2011229951 | A1 | 22-09-2011 | NONE | | |
| US 4436818 | A | 13-03-1984 | CH | 648348 A5 | 15-03-1985 |
| | | | EP | 0055753 A1 | 14-07-1982 |
| | | | IT | 1138835 B | 17-09-1986 |
| | | | US | 4436818 A | 13-03-1984 |
| | | | WO | 8200299 A1 | 04-02-1982 |
| US 4401441 | A | 30-08-1983 | NONE | | |
| DE 202009017017 | U1 | 03-02-2011 | DE 102010053242 A1 | | 22-06-2011 |
| | | | DE 202009017017 U1 | | 03-02-2011 |
| WO 9967176 | A1 | 29-12-1999 | AU | 4602899 A | 10-01-2000 |
| | | | CN | 1306497 A | 01-08-2001 |
| | | | WO | 9967176 A1 | 29-12-1999 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 4006382 A1